# FASCICULE DE BREVET EUROPEEN

(11) **EP 0 754 671 B1**
(45) Date de publication et mention de la délivrance du brevet: **31.03.1999**
(21) Numéro de dépôt: 96401590.3
(22) Date de dépôt: 17.07.1996
(51) Int. Cl.: C07C 57/04, C07C 51/48

(54) **Procédé de purification de l'acide acrylique obtenu par oxydation catalytique du propylène**
Verfahren zur Reinigung von durch katalytische Oxidation des Propylens gewonnener Acrylsäure
Process for the purification of acrylic acid obtained by the oxydation of propylene

(30) Priorité: 18.07.1995 FR 9508672
(43) Date de publication de la demande: 22.01.1997
(73) Titulaire: ELF ATOCHEM S.A., 92800 Puteaux, Hauts-de-Seine (FR)
(72) Inventeur: Fauconet, Michel, 57730 Valmont (FR); Esch, Marc, 57800 Freyming Merlebach (FR); Samuel, Yves, 57500 Saint-Avold (FR); Laurent, Denis, 57500 Saint-Avold (FR)
(74) Mandataire: Chaillot, Geneviève

(56) Documents cités:
- US-A- 3 689 541
- US-A- 3 868 417

## Description

La présente invention a pour objet un procédé de purification amélioré de l'acide acrylique.

La principale voie de synthèse d'acide acrylique utilisée industriellement aujourd'hui est l'oxydation catalytique du propylène, qui génère intermédiairement l'acroléine. Cette réaction, qui a lieu en phase gazeuse, génère un flux gazeux contenant principalement, outre l'acide acrylique, des gaz incondensables : propylène non converti, azote, monoxyde et dioxyde de carbone, des composés organiques "légers", c'est-à-dire dont le point d'ébullition est inférieur à celui de l'acide acrylique : vapeur d'eau, acroléine non convertie, impuretés fabriquées par des réactions secondaires, telles que le formaldéhyde, l'acide acétique, etc., et enfin, des composés lourds, c'est-à-dire dont le point d'ébullition est supérieur à celui de l'acide acrylique : anhydride maléique, furfuraldéhyde, benzaldéhyde, etc...

Les procédés de purification de ce gaz réactionnel, décrits dans la littérature, consistent à condenser ce mélange et à extraire les composés organiques par lavage à contre-courant à l'aide d'eau ou de solvants lourds.

Les procédés utilisant une absorption par l'eau présentent l'inconvénient d'extraire de manière peu sélective la quasi-totalité des produits organiques présents dans le mélange gazeux. La purification de la solution aqueuse ainsi constituée nécessite des séparations difficiles et coûteuses, par distillation et/ou extraction.

Dans le brevet français n 1 558 432, est décrit un procédé qui consiste à absorber les composés organiques contenus dans le gaz de réaction à l'aide d'esters d'acides aliphatiques ou aromatiques à points d'ébullition élevés, ou de phosphate de tributyle ou de tricrésyle. A l'issue de cette étape d'absorption, les légers (acroléine, formaldéhyde) sont éliminés en tête d'une première colonne de distillation, et une deuxième colonne de distillation permet d'obtenir, en tête, une solution aqueuse d'acide acrylique plus concentrée que dans l'art antérieur. Toutefois, la purification ultérieure de la solution obtenue, qui contient encore de l'acide acétique et de l'eau, nécessite encore des séparations coûteuses.

Le procédé décrit dans le brevet français n° 2 002 126 apporte une amélioration, grâce à l'utilisation d'un mélange de fractions de points d'ébullition élevés, récupéré en pied des colonnes de purification des esters fabriqués à partir de l'acide acrylique, contenant principalement des maléates, acides polyacryliques, polyacrylates. Ce procédé permet de débarrasser en une seule étape, en tête d'une colonne de distillation, la plus grande part des composés de points d'ébullition faibles, tels que l'acroléine, le formaldéhyde, l'eau et l'acide acétique. Toutefois, ce procédé de fabrication d'esters acryliques est mal adapté à la production d'acide acrylique pur, notamment à cause de la présence, dans le mélange d'acide acrylique brut initial, des dérivés d'estérification recyclés à l'étape d'absorption.

Une amélioration est apportée dans le procédé utilisant une extraction à l'aide d'un solvant lourd hydrophobe ou d'un mélange de solvants lourds hydrophobes, tel que décrit dans les brevets français n° 2 146 386 et allemand n° 4 308 087, qui permet d'obtenir, à l'issue de l'étape d'extraction, une solution anhydre et débarrassée d'une partie substantielle des produits organiques légers qui constituaient le mélange gazeux initial (acroléine, formaldéhyde, acide acétique), facilitant ainsi sensiblement la purification ultérieure de l'acide acrylique. Pour plus de clarté, on désignera par (c1) cette colonne d'extraction de l'acide acrylique par absorption des gaz de réaction à l'aide d'un solvant lourd ou d'un mélange de solvants lourds.

Selon ce dernier procédé, l'acide acrylique extrait, en solution dans le solvant lourd ou le mélange de solvants lourds, est d'abord, de manière optionnelle, débarrassé d'une partie de l'acide acétique et des composés "légers" résiduels (c'est-à-dire toutes les impuretés à point d'ébullition plus faible que l'acide acrylique) dans une colonne (c2), puis distillé dans une colonne (c3).

Toutefois, il est très difficile d'obtenir un produit totalement exempt de composés lourds (anhydride maléique, benzaldéhyde, furfuraldéhyde, traces de solvant(s)) en tête de cette colonne (c3), et, simultanément, en pied de colonne, un mélange de solvant(s) lourd(s) et de composés lourds, exempt d'acide acrylique résiduel.

On est donc contraint à choisir entre deux solutions qui présentent chacune ses inconvénients. Si l'on vise une teneur minimale d'acide acrylique en pied, on dégrade la qualité du flux de tête, principalement en composés plus lourds que l'acide acrylique, ce qui impose d'avoir recours à une purification supplémentaire coûteuse. A l'inverse, la recherche d'une qualité optimale de l'acide acrylique distillé en tête de colonne (c3) conduit à laisser passer de l'acide acrylique en pied. Dans ce cas, en l'absence d'un procédé approprié pour récupérer cet acide acrylique dans le flux de pied, comme c'est le cas, par exemple, dans le cadre de l'application du brevet français n 2 146 386, cette solution a pour conséquence une perte coûteuse de rendement de récupération de l'acide acrylique.

En effet, dans le brevet français mentionné n° 2 146 386, les étapes suivantes ont pour but de purifier le solvant lourd ou le mélange de solvants lourds, afin de le recycler à l'étape d'extraction de l'acide acrylique dans les gaz de réaction (colonne c1). En pied de colonne (c3), on obtient un mélange composé du solvant lourd ou du mélange de solvants lourds et des impuretés plus lourdes du procédé, à savoir :
- les impuretés "intermédiaires", c'est-à-dire celles dont le point d'ébullition est compris entre celui du solvant lourd et celui de l'acide acrylique : anhydride maléique, furfuraldéhyde, benzaldéhyde, etc...
- les impuretés "lourdes", c'est-à-dire à point d'ébullition supérieur celui du solvant lourd : oligomères acryliques estérifiés, polymères, inhibiteurs de polymérisation utilisés dans le procédé, etc...

A l'issue de cette première distillation, il est nécessaire d'éliminer les impuretés plus lourdes que l'acide acrylique, afin d'éviter leur accumulation lors du recyclage du solvant ou du mélange de solvants à l'étape d'extraction.

Comme il est décrit dans le brevet français mentionné n° 2 146 386, l'anhydride maléique peut être éliminé dans une colonne (c4), qui peut être soit une colonne de distillation (l'impureté est retirée en tête de cette colonne), soit une colonne d'extraction à l'eau (l'impureté est alors purgée en pied, dans le flux aqueux).

Dans les deux cas, l'acide acrylique présent en pied de colonne (c3) est perdu, soit en tête de la colonne de distillation (c4), soit dans l'eau rejetée de la colonne de lavage (c4). Ceci engendre une perte importante et coûteuse de rendement de récupération de l'acide acrylique.

L'extraction à l'eau présente l'inconvénient supplémentaire de générer un flux aqueux riche en pollution organique, qui nécessite un traitement d'élimination coûteux en énergie. De plus, l'élimination des impuretés "intermédiaires" autres que l'anhydride maléique est insuffisamment efficace, ce qui conduit à une accumulation de ces impuretés dans la boucle de recyclage du solvant. La conséquence est alors inévitablement l'entraînement de ces composés dans l'acide acrylique distillé.

L'élimination des impuretés "intermédiaires" en tête de colonne de distillation (c4) présente aussi le désavantage d'entraîner une perte sensible du ou des solvants lourds dans ce flux. Par ailleurs, les faibles teneurs d'eau présentes en pied de la colonne précédente (c3) de purification de l'acide acrylique se trouvent concentrées en tête de cette colonne d'élimination des produits "intermédiaires", et peuvent conduire à un phénomène gênant de précipitation d'acide maléique généré par réaction de la forme anhydride avec l'eau. Contrairement à l'anhydride, l'acide maléique est en effet très peu soluble dans ce milieu.

La Société déposante a maintenant mis au point une purification par distillation qui évite les inconvénients précédents. De manière surprenante, elle a en effet observé que, dans certaines conditions, on peut récupérer, dans une fraction soutirée latéralement dans une colonne de distillation (C4), un flux très riche en anhydride maléique, et, en tête de cette colonne, un flux très riche en acide acrylique, que l'on peut recycler à l'alimentation de la colonne précédente (C3) de purification de l'acide acrylique. De ce fait, il est possible de conduire la distillation dans la colonne (C3) de manière à obtenir en tête de cette colonne un flux très pur en acide acrylique, pratiquement exempt d'impuretés lourdes, en acceptant de laisser passer en pied une faible partie de l'acide acrylique présent dans l'alimentation. L'acide acrylique présent en pied de colonne (C3) n'est plus perdu, puisqu'il est récupéré en tête de la colonne suivante (C4) d'élimination des produits lourds "intermédiaires", et peut être recyclé à l'alimentation de la colonne précédente (C3). En outre, le flux soutiré latéralement dans cette colonne (C4) est particulièrement concentré en impuretés lourdes "intermédiaires" (principalement anhydride maléique, benzaldéhyde et furfuraldéhyde), ce qui permet d'éviter l'accumulation de ces impuretés dans la boucle de recyclage, et la perte de solvant(s) lourd(s) dans ce flux est très réduite.

La présente invention a donc pour objet un procédé de purification de l'acide acrylique obtenu par oxydation catalytique du propylène, extrait par lavage à contre-courant des gaz de réaction par au moins un solvant lourd hydrophobe dans une colonne d'extraction (C1), caractérisé par le fait :
- que l'on conduit, dans une colonne de distillation (C3), une distillation du flux obtenu en pied de la colonne d'extraction (C1), lequel contient le (ou les) solvant(s) lourd(s) d'extraction, l'acide acrylique recherché et des impuretés, principalement des impuretés dont les températures d'ébullition sont supérieures à celle de l'acide acrylique, ladite distillation étant effectuée dans des conditions telles que l'on obtienne un flux très pur d'acide acrylique en tête de ladite colonne (C3), en laissant passer de l'acide acrylique en pied ;
- que l'on envoie le flux de pied de la colonne (C3) en alimentation dans la partie inférieure d'une colonne de distillation (C4) de laquelle on soutire latéralement, sur un plateau situé entre l'alimentation et la tête de colonne, un flux riche en anhydride maléique et impuretés à températures d'ébullition situées entre celle de l'acide acrylique et celle dudit solvant lourd ou du plus léger desdits solvants lourds utilisés en mélange ;
- que l'on distille, en tête de la colonne (C4), un flux riche en acide acrylique ; et
- qu'on récupère, en pied de ladite colonne (C4), un flux contenant dudit (ou desdits) solvant(s) lourd(s) et des impuretés lourdes dont les températures d'ébullition sont supérieures à celle dudit solvant lourd ou du plus léger desdits solvants lourds utilisés en mélange, flux que l'on recycle en tête de la colonne (C1) pour l'extraction de l'acide acrylique contenu dans les gaz de réaction.

Avant d'adresser à la colonne (C3) le flux obtenu en pied de la colonne (C1), on peut avantageusement débarrasser ledit flux d'une partie de ses impuretés résiduelles légères, telles que l'acide acétique, en tête d'une colonne de distillation (C2).

Conformément à des modes de réalisation particuliers du procédé selon la présente invention :
- on envoie le flux obtenu en pied de la colonne (C1), le cas échéant en pied de la colonne (C2), sur un plateau situé dans la moitié inférieure de la colonne (C3), et on recherche le point de fonctionnement de ladite colonne (C3) de façon à obtenir :
   - en tête, un flux composé :
      - majoritairement, soit au moins 95% en poids, d'acide acrylique ;
      - le reste étant constitué par les composés lourds : anhydride maléique, furfuraldéhyde, benzaldéhyde et traces du (ou des) solvant(s) lourd(s) d'extraction ; et
   - en pied, un flux composé de :
      - majoritairement, soit au moins 95% en poids, du (ou des) solvant(s) lourd(s) et des impuretés lourdes ;
      - le reste étant constitué par de l'acide acrylique ;
- on soutire latéralement de la colonne (C4) le flux riche en anhydride maléique et impuretés lourdes sur un plateau intermédiaire, situé au-dessus de l'alimentation entre le quart inférieur et le quart supérieur de cette colonne, à une température choisie de façon à obtenir un flux d'une concentration au moins égale à 20% en poids en impuretés à températures d'ébullition comprises entre celle de l'acide acrylique et celle du solvant ou du plus léger des solvants utilisés en mélange ;
- on envoie le flux distillé en tête de la colonne (C4), qui contient :
   - majoritairement, soit au moins 90% en poids, d'acide acrylique ;
   - le reste étant constitué par des impuretés à températures d'ébullition supérieures ;
   dans la colonne (C3), au niveau de l'alimentation principale de cette colonne ou, de manière avantageuse, à un niveau situé au-dessus de cette alimentation ; et
- avant de recycler en tête de la colonne (C1) le flux obtenu en pied de colonne (C4), on débarrasse ledit flux ou une partie dudit flux de ses impuretés lourdes à températures d'ébullition supérieures à celle du ou des solvants, par exemple par des techniques de distillation ou extraction à l'aide d'un solvant, utilisées éventuellement en complément d'un traitement thermique de dissociation mettant en jeu ou non un catalyseur.

Conformément à des modes de réalisation particuliers de la présente invention :
- on conduit la distillation dans la colonne (C3) sous une pression de 2,66 x 10³ - 3,33 x 10⁴ Pa (20 - 250 mmHg), à une température de tête de 40 - 120°C et à une température de pied de 120 - 230'C ;
- on conduit la distillation dans la colonne (C4) sous une pression de 2,66 x 10³ - 3,33 x 10⁴ Pa (20 - 250 mmHg), à une température de tête de 40 - 120°C, à une température de pied de 120 - 230°C et à une température de soutirage latéral de 40 - 180°C ;
- on conduit la distillation dans la colonne (C2) sous une pression de 2,66 x 10³ - 3,33 x 10⁴ Pa (20 - 250 mmHg), à une température de tête de 30 - 110°C et à une température de pied de 70 - 170°C.

On peut également préciser les points suivants :
(1) Les colonnes à distiller seront équipées de préférence des dispositifs connus pour la distillation sous vide de produits polymérisables dans les sections traitant des quantités significatives d'AA ; les plateaux perforés sans déversoir sont particulièrement recommandés. Les viroles pourront être maintenues par tout dispositif approprié à une température suffisante pour éviter les points froids (qui amèneraient une condensation de produit non stabilisé et là-même des risques de polymérisation).
(2) Des inhibiteurs de polymérisation doivent être introduits dans les colonnes à distiller de préférence en présence d'oxygène ; les différents inhibiteurs connus pour leur efficacité d'inhibition de la polymérisation de l'acide acrylique tels que les dérivés phénoliques, la phénothiazine et ses dérivés, les thiocarbamates métalliques, les composés à groupements nitroso, les dérivés de la paraphénylènediamine, les quinones, peuvent convenir.
(3) Dans le descriptif précédent, le flux gazeux issu de (C1) peut être partiellement recyclé à la zone réactionnelle. Dans tous les cas toutefois, une partie au moins de ce flux doit être éliminé du système : si cette partie doit être rejetée à l'atmosphère, elle doit être purifiée soit par oxydation catalytique, soit par passage dans une chambre de combustion.
(4) Le flux sortant en tête de la colonne (C2), riche en acide acétique, peut avantageusement être introduit en un point approprié de la colonne (C1), ce qui permettra de récupérer une fraction notable de l'acide acrylique contenu (et d'éliminer l'acide acétique).
(5) Dans le cas où on utilise un inhibiteur ou une combinaison d'inhibiteurs légers, celui-ci ou ceux-ci pourront être éliminés, au moins partiellement, dans le flux de soutirage latéral de la colonne (C4).

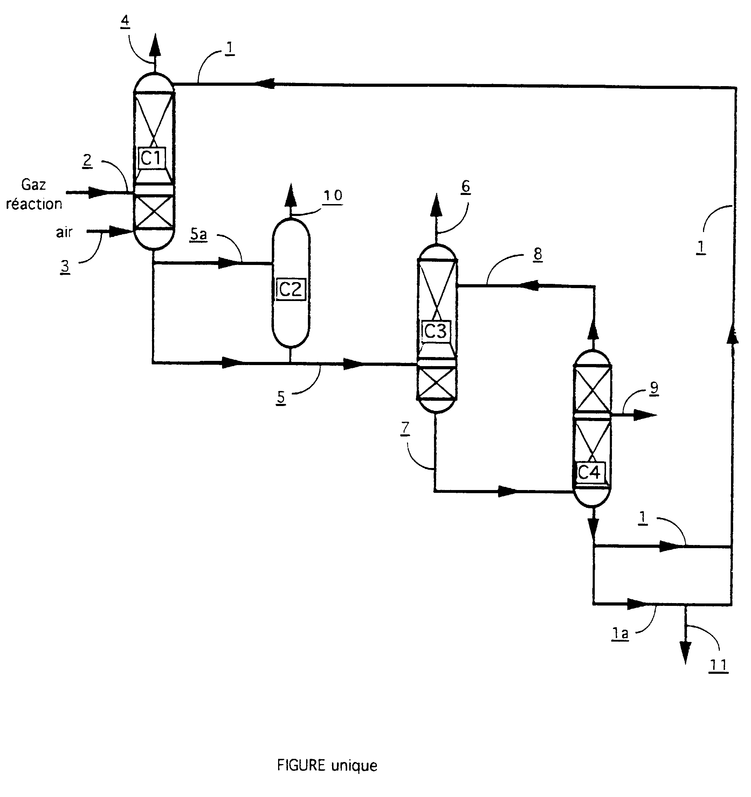

Les Exemples suivants illustrent la présente invention sans toutefois en limiter la portée. Dans ces exemples, les pourcentages sont donnés en poids. On a utilisé des colonnes de distillation qui sont installées selon le schéma de la figure unique du dessin annexé et dont les caractéristiques sont indiquées ci-après. L'alimentation des colonnes pourra éventuellement être réchauffée à l'aide d'un échangeur. Une injection d'oxygène est faite dans les colonnes. Dans le texte relatif à la description de ces exemples, on numérote les plateaux des colonnes à partir de la tête de colonne (plateau 0) jusqu'en pied de colonne (plateau n).

### · Colonne C1 :

Cette colonne, de 3 m de hauteur et de 38 mm de diamètre, garnie d'éléments de remplissage de type "multiknit", est alimentée :
- en tête, par un courant (1) de solvant d'absorption, constitué par un mélange de 25% de diphényle et 75% de diphényléther, pur ou récupéré en pied de colonne (C4), éventuellement après une opération de déconcentration des impuretés ;
- au premier quart inférieur, par le courant (2) de gaz réactionnel d'oxydation catalytique du propylène, préalablement refroidi à 90°C ;
- en pied, par de l'air (3) utilisé pour effectuer un stripping des composés légers.

En tête de colonne (C1), on obtient un flux (4) riche en composés légers (gaz incondensables, acroléine, formaldéhyde, eau, ainsi qu'une partie de l'acide acétique). En pied de colonne (C1), le flux (5) récupéré constitue une solution dans le solvant d'absorption de l'acide acrylique avec l'acide acétique résiduel et les constituants lourds de ce flux : anhydride maléique, furfuraldéhyde, benzaldéhyde, composés d'addition sur l'acide acrylique, inhibiteurs.

### · Colonne C3 :

Cette colonne, garnie d'éléments de remplissage de type "multiknit", d'efficacité 18 plateaux théoriques, est alimentée au premier tiers inférieur par un mélange caractéristique du flux (5) obtenu en pied de colonne (C1). Une partie du distillat (6) récupéré en tête de colonne après condensation est envoyée à nouveau en haut de colonne pour assurer le reflux. La colonne est équipée en partie inférieure d'un bouilleur chauffé électriquement.

### · Colonne C4 :

Cette colonne est constituée de 9 plateaux réels, d'efficacité 5 plateaux théoriques, chaque plateau étant muni d'orifices et d'une surverse. L'alimentation, par un mélange caractéristique du flux (7) obtenu en pied de colonne (C3), est préalablement préchauffée à travers un échangeur, puis envoyée en pied de cette colonne, au niveau du bouilleur à thermosiphon chauffé par résistances électriques. Une partie du distillat (8) récupéré en tête de colonne après condensation est envoyée à nouveau en haut de colonne pour assurer le reflux. Au niveau du cinquième plateau, un système permet de soutirer (en 9) une partie de la phase liquide condensée. Ce système est muni d'une vanne dont l'ouverture est commandée automatiquement lorsque la température mesurée sur ce plateau atteint la consigne de température fixée.

### EXEMPLE 1

Dans la colonne de distillation (C3), fonctionnant sous une pression de 0,133 bar (100 mm Hg), on envoie 1150 g/h d'un flux (5) obtenu en pied de colonne d'extraction (C1), comprenant :
- 90 % du solvant d'absorption lourd (mélange de 25% diphényle et 75% diphényléther) ;
- 9,31% d'acide acrylique ;
- 0,07% d'anhydride maléique.

En tête de colonne (C3), on renvoie une partie du flux distillé (6), de façon à assurer un taux de reflux/soutirage de 1/1. On envoie également, en tête de cette colonne (C3), à un débit de 10 g/h, un mélange d'acide acrylique à 1,5% d'hydroquinone, qui joue le rôle d'inhibiteur de polymérisation. On fait varier la température au niveau du bouilleur situé en pied de colonne.

Les résultats sont résumés dans le tableau ci-dessous :

| Température pied | 160°C | 170°C | 182°C |
|---|---|---|---|
| % récupération AA | 89,3% | 94,3% | 99,8% |
| solvant tête (C3) | <0,001% | 0,02% | 0,112% |
| MAA tête (C3) | 0,006% | 0,212% | 0,529% |
| AA pied (C3) | 1,080% | 0,595% | 0,021% |
| AA : acide acrylique | | | |
| MAA : anhydride maléique | | | |

Cet exemple illustre la difficulté d'obtenir à la fois une bonne qualité d'acide acrylique, exempte de composés lourds, en tête de colonne (C3), et une faible teneur en acide acrylique en pied (ou un bon taux de récupération en tête). Une qualité d'acide acrylique dépourvue de composés lourds ne peut être obtenue qu'en acceptant de laisser passer une partie non négligeable d'acide acrylique en pied de colonne.

### EXEMPLE 2

En fond de colonne (C4), qui fonctionne sous une pression de 0,133 bar (100 mm Hg), on envoie 1000 g/h d'un mélange (7) récupéré en pied de colonne (C3), comprenant :
- 0,69 % d'acide acrylique ;
- 0,06 % d'anhydride maléique ;
- 0,005% de benzaldéhyde ;
- 0,003% de furfuraldéhyde ;
- 0,04 % d'hydroquinone ;
- le reste étant constitué du solvant lourd d'absorption (mélange de diphényle et diphényléther) et de traces de composés lourds d'addition sur la double liaison de l'acide acrylique.

Ce mélange (7) est préchauffé à une température de 180°C avant d'être envoyé dans le bouilleur de la colonne (C4). La température dans le bouilleur est régulée à 182°C. La température de consigne de soutirage du flux (9) riche en composés lourds intermédiaires, au niveau du cinquième plateau, est fixée à 135°C. On distille en tête de colonne (C4), à une température de 84°C, un flux (8) (6,4 g/h) constitué de :
- 97,1 % d'acide acrylique ;
- 2,49% d'anhydride maléique ;
- 0,36% de benzaldéhyde ; et
- 0,07% de solvant d'absorption.

Le flux (9) soutiré latéralement au niveau du cinquième plateau (0,5 g/h) est composé de :
- 62 % d'anhydride maléique ;
- 29 % de solvant lourd d'absorption ;
- 5,3% d'acide acrylique ;
- 3,1% de benzaldéhyde ; et
- 1,2% de furfuraldéhyde.

Enfin, en pied de colonne (C4), on soutire un mélange (1) constitué essentiellement du solvant lourd d'absorption et des composés lourds d'addition sur l'acide acrylique, ainsi que 0,02% d'acide acrylique, 0,016% d'anhydride maléique et 0,038% d'hydroquinone.

Dans ces conditions, on voit que, si on renvoie le flux (8) obtenu en tête de colonne (C4) à l'alimentation de la colonne précédente (C3), la perte d'acide acrylique est limitée à la quantité de cet acide qui est soutirée latéralement, soit inférieure à 0,5%. En l'absence du procédé qui fait l'objet de la présente invention, la quasi-totalité de l'acide acrylique présent dans l'alimentation de cette colonne (C4) serait perdue.

En outre, le procédé selon l'invention permet de déconcentrer suffisamment en impuretés intermédiaires le flux (1) de solvant d'absorption à recycler, de façon à éviter l'accumulation de ces impuretés dans la boucle. Le taux de réduction de ces impuretés, soutirées latéralement, est de 48% pour l'anhydride maléique, 30% pour le benzaldéhyde et 20% pour le furfuraldéhyde.

### EXEMPLE 3 (comparatif)

En pied de colonne (C4), qui fonctionne sous une pression de 0,133 bar (100 mm Hg), on envoie 1000 g/h d'un flux préchauffé à 180°C, récupéré en pied de colonne précédente (C3), composé de :
- 1,6 % d'acide acrylique ;
- 0,035% d'anhydride maléique ;
- 0,006% de benzaldéhyde ;
- le reste étant constitué du solvant d'absorption (25% de diphényle, 75% de diphényléther) et des composés lourds (dérivés d'addition sur l'acide acrylique, inhibiteurs, ...).

La température en pied de colonne est de 182°C. Contrairement à l'exemple précédent, on ne soutire aucun flux sur un plateau intermédiaire.

Le distillat prélevé en tête (16,6 g/h), à une température de 85°C, est composé de :
- 97,8 % d'acide acrylique ;
- 1,41% d'anhydride maléique ;
- 0,22% de benzaldéhyde ; et
- 0,11% de solvant d'absorption.

Le flux récupéré en pied de colonne (C4) contient 0,047% d'acide acrylique, 0,01% d'anhydride maléique et 0,002% de benzaldéhyde, le reste étant constitué principalement par le solvant d'absorption et les composés lourds d'addition sur l'acide acrylique et les inhibiteurs.

Dans ces conditions, la perte d'acide acrylique dans le flux récupéré en tête de colonne par rapport à l'acide acrylique présent dans les flux sortants de la colonne (C4), est de 97%.

### EXEMPLE 4

Dans cet exemple, l'ensemble de la boucle de distillation (C1) - (C3) - (C4) est mise en oeuvre successivement, avec des recyclages de produits obtenus lors d'un passage préalable sur l'ensemble de distillation, à savoir du solvant (1) récupéré en pied de la colonne (C4) et du flux (8) distillé en tête de colonne (C4), respectivement comme solvant d'absorption en tête de colonne (C1) et comme complément en alimentation de la colonne (C3).

La colonne (C1) est alimentée :
- en tête, par un mélange (1) de solvant d'absorption récupéré en pied de colonne (C4) (2700 g/h), dans lequel on analyse de faibles teneurs d'acide acrylique (0,047%), d'anhydride maléique (0,01%), de benzaldéhyde (0,002%) et d'hydroquinone (0,05%) ;
- au niveau du premier quart inférieur, par les gaz (2) de réaction d'oxydation catalytique du propylène (2160 g/h), préalablement refroidis à 90°C ; et
- en pied (3), par de l'air (600 1/h).

En pied de cette colonne (C1), on obtient un flux (5) constitué par :
- 8,66 % d'acide acrylique ;
- 0,064% d'anhydride maléique ;
- 0,006% de benzaldéhyde ;
- 0,005% de furfuraldéhyde ;
- 0,055% d'hydroquinone ;
- le reste étant constitué principalement par le solvant d'absorption.

Ce mélange (5) est complété par le flux (8) obtenu en tête de la colonne (C4) lors d'un passage préalable, composé d'acide acrylique (97,8%), d'anhydride maléique (1,4%), de benzaldéhyde (0,22%), de furfuraldéhyde (0,12%), de solvant (0,11%). Ce mélange est envoyé (1231 g/h) en alimentation de colonne (C3), qui fonctionne sous une pression de 0,133 bar (100 mm Hg). La température au niveau de bouilleur est fixée à 165°C. En tête de colonne (C3), on renvoie une partie du flux distillé (6), de façon à assurer un taux de reflux/soutirage de 1/1. On envoie également, en tête de cette colonne, à un débit de 9,8 g/h, un mélange d'acide acrylique à 1,5% d'hydroquinone. Le produit (6) qui distille en tête à une température de 83°C est essentiellement de l'acide acrylique, contient en outre de faibles teneurs en anhydride maléique (0,16%), benzaldéhyde (0,01%), furfuraldéhyde (0,02%) et est exempt de solvant d'absorption.

Le flux (7) récupéré en pied de colonne (C3), qui contient :
- 1,03 % d'acide acrylique ;
- 0,09% d'anhydride maléique ;
- 0,007% de benzaldéhyde ;
- 0,003% de furfuraldéhyde ;
- 0,073% d'hydroquinone ;
- le reste étant constitué par le solvant d'absorption et les composés lourds d'addition sur l'acide acrylique,
est envoyé (1000 g/h) en pied de colonne (C4), qui fonctionne sous une pression de 0,133 bar (100 mm Hg), à une température de 180°C. La température au niveau du bouilleur est régulée à 182°C, et la température de consigne de soutirage au niveau du cinquième plateau est fixée à 135°C. Le produit 8, distillé en tête de colonne (9,3 g/h), destiné à être renvoyé en alimentation de colonne (C3), est composé de :
- 98 % d'acide acrylique ;
- 1,45% d'anhydride maléique ;
- 0,17% de benzaldéhyde ;
- 0,15% de furfuraldéhyde ; et
- 0,08% de solvant d'absorption.

Le flux 9 soutiré en soutirage latéral (1,31 g/h) contient :
- 12,94% d'acide acrylique ;
- 36,4 % d'anhydride maléique ;
- 47,8 % de solvant d'absorption ;
- 1,73% de benzaldéhyde ; et
- 0,83% de furfuraldéhyde.
Enfin, le mélange (1) obtenu en pied de colonne (C4), destiné à être recyclé en tête de colonne (C1), est principalement constitué du solvant d'absorption, des produits lourds d'addition sur l'acide acrylique et de l'hydroquinone (0,074%), avec de faibles teneurs d'acide acrylique (0,04%) et anhydride maléique (0,02%), et des traces de benzaldéhyde (0,002%) et de furfuraldéhyde (teneur inférieure à 0,001%).

Dans ces conditions, la perte d'acide acrylique dans le soutirage latéral n'est que de 1,6%, tandis que le taux de réduction des impuretés intermédiaires dans le flux de solvant d'absorption à recycler est de 80% pour l'anhydride maléique et 79% pour le benzaldéhyde.

Comme cela a déjà été indiqué, on peut adresser le flux 5 (selon flèche 5a) à une colonne de distillation (C2) pour le débarrasser (en 10) de ses impuretés résiduelles légères.

Avant de recycler le flux 1 à la colonne (C1), on peut également le débarrasser (selon flèche 1a) de ses impuretés lourdes (en 11), par des techniques connues, telles que celles définies ci-dessus.

## Revendications

1. Procédé de purification de l'acide acrylique obtenu par oxydation catalytique du propylène, extrait par lavage à contre-courant des gaz de réaction par au moins un solvant lourd hydrophobe dans une colonne d'extraction (C1), caractérisé par le fait :
- que l'on conduit, dans une colonne de distillation (C3), une distillation du flux (5) obtenu en pied de la colonne d'extraction (C1), lequel contient le (ou les) solvant(s) lourd(s) d'extraction, l'acide acrylique recherché et des impuretés, principalement des impuretés dont les températures d'ébullition sont supérieures à celle de l'acide acrylique, ladite distillation étant effectuée dans des conditions telles que l'on obtienne un flux très pur d'acide acrylique (6) en tête de ladite colonne (C3), en laissant passer de l'acide acrylique en pied (7) ;
- que l'on envoie le flux de pied (7) de la colonne (C3) en alimentation dans la partie inférieure d'une colonne de distillation (C4) de laquelle on soutire latéralement, sur un plateau situé entre l'alimentation et la tête de colonne, un flux (9) riche en anhydride maléique et impuretés à températures d'ébullition situées entre celle de l'acide acrylique et celle dudit solvant lourd ou du plus léger desdits solvants lourds utilisés en mélange ;
- que l'on distille, en tête de la colonne (C4), un flux (8) riche en acide acrylique ; et
- qu'on récupère, en pied de ladite colonne (C4), un flux (1) contenant dudit (ou desdits) solvant(s) lourd(s) d'extraction et des impuretés lourdes, dont les températures d'ébullition sont supérieures à celle dudit solvant lourd ou du plus léger desdits solvants lourds utilisés en mélange, flux que l'on recycle en tête de la colonne (C1) pour l'extraction de l'acide acrylique contenu dans les gaz de réaction.

2. Procédé selon la revendication 1, caractérisé par le fait qu'on débarrasse le flux (5) obtenu en pied de la colonne (C1) d'une partie de ses impuretés résiduelles légères (10), telles que l'acide acétique, en tête d'une colonne de distillation (C2).

3. Procédé selon l'une des revendications 1 et 2, caractérisé par le fait que l'on envoie le flux (5) obtenu en pied de la colonne (C1), le cas échéant en pied de la colonne (C2), sur un plateau situé dans la moitié inférieure de la colonne (C3), et que l'on recherche le point de fonctionnement de ladite colonne (C3) de façon à obtenir :
- en tête, un flux (6) composé :
- majoritairement, soit au moins 95% en poids, d'acide acrylique ;
- le reste étant constitué par les composés lourds : anhydride maléique, furfuraldéhyde, benzaldéhyde et traces du (ou des) solvant(s) lourd(s) d'extraction ; et
- en pied, un flux (7) composé de :
- majoritairement, soit au moins 95% en poids, du (ou des) solvant(s) lourd(s) et des impuretés lourdes ;
- le reste étant constitué par de l'acide acrylique.

4. Procédé selon l'une des revendications 1 à 3, caractérisé par le fait que l'on soutire latéralement de la colonne (C4) le flux (9) riche en anhydride maléique et impuretés lourdes sur un plateau intermédiaire, situé au-dessus de l'alimentation entre le quart inférieur et le quart supérieur de cette colonne, à une température choisie de façon à obtenir un flux (9) d'une concentration au moins égale à 20% en poids en impuretés à températures d'ébullition comprises entre celle de l'acide acrylique et celle du solvant d'extraction ou du plus léger des solvants utilisés en mélange.

5. Procédé selon l'une des revendications 1 à 4, caractérisé par le fait que l'on envoie le flux (8) distillé en tête de la colonne (C4), qui contient :
- majoritairement, soit au moins 90% en poids, d'acide acrylique ;
- le reste étant constitué par des impuretés à températures d'ébullition supérieures,
à la colonne (C3), au niveau de l'alimentation principale de cette colonne ou à un niveau situé au-dessus de cette alimentation.

6. Procédé selon l'une des revendications 1 à 5, caractérisé par le fait qu'avant de recycler en tête de la colonne (C1) le flux (1) obtenu en pied de colonne (C4), on débarrasse ledit flux ou une partie dudit flux (en 11) de ses impuretés lourdes à températures d'ébullition supérieures à celle du ou des solvants, par exemple par des techniques de distillation ou extraction à l'aide d'un solvant, utilisées éventuellement en complément d'un traitement thermique de dissociation mettant en jeu ou non un catalyseur.

7. Procédé selon l'une des revendications 1 à 6, caractérisé par le fait qu'on conduit la distillation dans la colonne (C3) sous une pression de 2,66 x 10³ - 3,33 x 10⁴ Pa, à une température de tête de 40 - 120°C et à une température de pied de 120 - 230°C.

8. Procédé selon l'une des revendications 1 à 7, caractérisé par le fait qu'on conduit la distillation dans la colonne (C4) sous une pression de 2,66 x 10³ - 3,33 x 10⁴ Pa, à une température de tête de 40 - 120°C, à une température de pied de 120 - 230°C, et à une température de soutirage latéral de 40 - 180°C.

9. Procédé selon l'une des revendications 2 à 8, caractérisé par le fait qu'on conduit la distillation dans la colonne (C2) sous une pression de 2,66 x 10³ - 3,33 x 10⁴ Pa, à une température de tête de 30 - 110°C et à une température de pied de 70 - 170°C.

## Patentansprüche

1. Verfahren zur Reinigung von Acrylsäure, die durch katalytische Oxidation von Propylen hergestellt und durch Waschen der Reaktionsgase im Gegenstrom mit mindestens einem hydrophoben, hochsiedenden Lösungsmittel in einer Extraktionskolonne (C1) extrahiert wurde,
dadurch gekennzeichnet, daß:
- in einer Destillationskolonne (C3) eine Destillation des Flusses (5) durchgeführt wird, der am Fuß der Extraktionskolonne (C1) erhalten wird und der das hochsiedende Lösungsmittel oder die hochsiedenden Lösungsmittel der Extraktion, die gewünschte Acrylsäure und Verunreinigungen, hauptsächlich Verunreinigungen, deren Siedetemperatur über der Siedetemperatur von Acrylsäure liegt, enthält, wobei die Destillation unter solchen Bedingungen durchgeführt wird, daß am Kopf der Kolonne (C3) ein sehr reiner Fluß von Acrylsäure (6) erhalten wird, indem Acrylsäure am Fuß (7) übergehen gelassen wird;
- der Fluß am Fuß (7) der Kolonne (C3) in den unteren Teil einer Destillationskolonne (C4) zugeführt wird, aus der seitlich an einem Boden zwischen Zuführung und Kolonnenkopf ein Fluß (9) entnommen wird, der einen hohen Gehalt an Maleinsäureanhydrid und Verunreinigungen mit einem Siedepunkt, der zwischen dem Siedepunkt von Acrylsäure und dem Siedepunkt des hochsiedenden Lösungsmittels oder des am niedrigsten siedenden Lösungsmittels der im Gemisch verwendeten hochsiedenden Lösungsmittel liegt, aufweist;
- am Kopf der Kolonne (C4) ein Fluß (8), der einen hohen Gehalt an Acrylsäure aufweist, abdestilliert wird;
- am Fuß der Kolonne (C4) ein Fluß (1) gewonnen wird, der das hochsiedende Lösungsmittel oder die hochsiedenden Lösungsmittel der Extraktion und die hochsiedenden Verunreinigungen, deren Siedepunkt über dem Siedepunkt des hochsiedenden Lösungsmittels oder des am niedrigsten siedenden Lösungsmittels der im Gemisch verwendeten hochsiedenden Lösungsmittel liegt, enthält, wobei dieser Fluß zum Kopf der Kolonne (C1) zur Extraktion der in den Reaktionsgasen enthaltenen Acrylsäure zurückgeführt wird.

2. Verfahren nach Anspruch 1, dadurch gekennzeichnet, daß ein Teil der restlichen niedrigsiedenden Verunreinigungen (10), wie beispielsweise Essigsäure, aus dem am Fuß der Kolonne (C1) erhaltenen Fluß (5) am Kopf einer Destillationskolonne (C2) entfernt werden.

3. Verfahren nach einem der Ansprüche 1 und 2, dadurch gekennzeichnet, daß der am Fuß der Kolonne (C1) und gegebenenfalls am Fuß der Kolonne (C2) erhaltene Fluß (5) zu einem Boden geleitet wird, der sich in der unteren Hälfte der Kolonne (C3) befindet, und der Funktionspunkt der Kolonne (C3) so eingestellt wird, daß erhalten werden:
- am Kopf ein Fluß (6) aus:
- hauptsächlich, d.h. mindestens 95 Gew.-% Acrylsäure;
- wobei die restlichen Bestandteile hochsiedende Verbindungen sind: Maleinsäureanhydrid, Furfurylaldehyd, Benzaldehyd und Spuren des oder der hochsiedenden Lösungsmittel(s) der Extraktion; und
- am Fuß ein Fluß (7) aus:
- hauptsächlich, d.h. mindestens 95 Gew.-% des oder der hochsiedenden Lösungsmittel(s) und hochsiedenden Verbindungen;
- wobei der Rest aus Acrylsäure besteht.

4. Verfahren nach einem der Ansprüche 1 bis 3, dadurch gekennzeichnet, daß seitlich an der Kolonne (C4) der Fluß (9), der einen hohen Gehalt an Maleinsäureanhydrid und hochsiedenden Verunreinigungen aufweist, an einem Zwischenboden, der sich über der Zuleitung zwischen dem unteren Viertel und dem oberen Viertel der Kolonne befindet, bei einer Temperatur entnommen wird, die so ausgewählt ist, daß ein Fluß (9) erhalten wird, der eine Konzentration von mindestens 20 Gew.-% Verunreinigungen mit einer Siedetemperatur, die zwischen der Siedetemperatur von Acrylsäure und der Siedetemperatur des Lösungsmittels der Extraktion oder des am niedrigsten siedenden Lösungsmittels der im Gemisch verwendeten Lösungsmittel liegt, aufweist.

5. Verfahren nach einem der Ansprüche 1 bis 4, dadurch gekennzeichnet, daß der Fluß (8), der am Kopf der Kolonne (C4) abdestilliert wird und der enthält:
- hauptsächlich, d.h. mindestens 90 Gew.-% Acrylsäure;
- wobei die restlichen Bestandteile Verunreinigungen mit höheren Siedepunkten sind;
in Höhe der Hauptzuleitung oder in einer Höhe, die über dieser Zuleitung liegt, zu der Kolonne (C3) geführt wird.

6. Verfahren nach einem der Ansprüche 1 bis 5, dadurch gekennzeichnet, daß der am Fuß der Kolonne (C4) erhaltene Fluß (1) oder eine Teil dieses Flusses, bevor er zum Kopf der Kolonne (C1) zurückgeführt wird, von den hochsiedenden Verunreinigungen befreit wird (bei 11), die einen Siedepunkt aufweisen, der über dem Siedepunkt des Lösungsmittels oder der Lösungsmittel liegt, beispielsweise durch Verfahren der Destillation oder Extraktion mit einem Lösungsmittel, die gegebenenfalls zusätzlich zu einer thermischen Dissoziationsbehandlung, die gegebenenfalls unter Verwendung eines Katalysators durchgeführt wird, angewandt werden.

7. Verfahren nach einem der Ansprüche 1 bis 6, dadurch gekennzeichnet, daß die Destillation in der Kolonne (C3) bei einem Druck von 2,66 · 10³ bis 3,33 · 10⁴ Pa, einer Temperatur am Kopf von 40 bis 120 °C und einer Temperatur am Fuß von 120 °C bis 230 °C durchgeführt wird.

8. Verfahren nach einem der Ansprüche 1 bis 7, dadurch gekennzeichnet, daß die Destillation in der Kolonne (C4) bei einem Druck von 2,66 · 10³ bis 3,33 · 10⁴ Pa, einer Temperatur am Kopf von 40 bis 120 °C, einer Temperatur am Fuß von 120 °C bis 230 °C und einer Temperatur bei der seitlichen Entnahme von 40 bis 180 °C durchgeführt wird.

9. Verfahren nach einem der Ansprüche 2 bis 8, dadurch gekennzeichnet, daß die Destillation in der Kolonne (C2) bei einem Druck von 2,66 · 10³ bis 3,33 · 10⁴ Pa, einer Temperatur am Kopf von 30 bis 110 °C und einer Temperatur am Fuß von 70 °C bis 170 °C durchgeführt wird.

## Claims

1. Process for the purification of acrylic acid obtained by catalytic oxidation of propylene, extracted by countercurrent washing of the reaction gases with at least one hydrophobic heavy solvent in an extraction column (C1), characterized in that:
- a distillation of the stream (5) obtained at the bottom of the extraction column (C1), which contains the heavy extraction solvent(s), the required acrylic acid and impurities, chiefly impurities whose boiling temperatures are higher than that of acrylic acid, is conducted in a distillation column (C3), the said distillation being performed in conditions such that a stream (6) which is very pure in respect of acrylic acid is obtained at the top of the said column (C3), allowing acrylic acid to pass at the bottom (7);
- the stream from the bottom (7) of the column (C3) is conveyed as feed into the lower part of a distillation column (C4) from which there is drawn off sideways, on a tray situated between the feed and the top of the column, a stream (9) which is rich in maleic anhydride and impurities with boiling temperatures situated between that of acrylic acid and that of the said heavy solvent or of the lightest of the said heavy solvents employed as a mixture;
- a stream (8) rich in acrylic acid is distilled at the top of the column (C4); and
- a stream (1) containing the said heavy extraction solvent(s) and heavy impurities whose boiling temperatures are higher than that of the said heavy solvent or of the lightest of the said heavy solvents employed as a mixture is recovered at the bottom of the said column (C4), this stream being recycled to the top of the column (C1) for the extraction of the acrylic acid present in the reaction gases.

2. Process according to Claim 1, characterized in that the stream (5) obtained at the bottom of the column (C1) is freed from a proportion of its light residual impurities (10), such as acetic acid, at the top of a distillation column (C2).

3. Process according to either of Claims 1 and 2, characterized in that the stream (5) obtained at the bottom of the column (C1), if appropriate at the bottom of the column (C2), is conveyed onto a tray situated in the lower half of the column (C3), and in that the operating point of the said column (C3) is searched for so as to obtain:
- at the top, a stream (6) composed:
- predominantly, that is at least 95 % by weight, of acrylic acid;
- the remainder consisting of the heavy compounds: maleic anhydride, furfuraldehyde, benzaldehyde and traces of the heavy extraction solvent(s); and
- at the bottom, a stream (7) composed:
- predominantly, that is at least 95 % by weight, of the heavy solvent(s) and of the heavy impurities;
- the remainder consisting of acrylic acid.

4. Process according to one of Claims 1 to 3, characterized in that the stream (9) which is rich in maleic anhydride and heavy impurities is drawn off sideways from the column (C4) on an intermediate tray situated above the feed between the lower quarter and the upper quarter of this column, at a temperature chosen so as to obtain a stream (9) with a concentration of at least 20 % by weight in respect of impurities with boiling temperatures between that of acrylic acid and that of the extraction solvent or of the lightest of the solvents employed as a mixture.

5. Process according to one of Claims 1 to 4, characterized in that the stream (8) distilled at the top of the column (C4), which contains:
- predominantly, that is at least 90 % by weight, acrylic acid;
- the remainder consisting of impurities of higher boiling temperatures,
is conveyed into the column (C3), at the main feed level of this column or at a level situated above this feed.

6. Process according to one of Claims 1 to 5, characterized in that, before recycling the stream (1) obtained at the bottom of column (C4) to the top of the column (C1), the said stream or a proportion of the said stream is freed (in 11) from its heavy impurities with boiling temperatures higher than that of the solvent(s), for example by distillation techniques or extraction with the aid of a solvent, which are employed optionally as a supplement to a dissociation heat treatment optionally involving a catalyst.

7. Process according to one of Claims 1 to 6, characterized in that the distillation in the column (C3) is conducted at a pressure of 2.66 × 10³ - 3.33 × 10⁴ Pa, at a top temperature of 40 - 120°C and at a bottom temperature of 120 - 230°C.

8. Process according to one of Claims 1 to 7, characterized in that the distillation in the column (C4) is conducted at a pressure of 2.66 × 10³ - 3.33 × 10⁴ Pa, at a top temperature of 40 - 120°C, at a bottom temperature of 120 - 230°C and at a side draw-off temperature of 40 - 180°C.

9. Process according to one of Claims 2 to 8, characterized in that the distillation in the column (C2) is conducted at a pressure of 2.66 × 10³ - 3.33 × 10⁴ Pa, at a top temperature of 30 - 110°C and at a bottom temperature of 70 - 170°C.
